# FASCICULE DE BREVET EUROPEEN

(11) **EP 1 523 294 B1**
(45) Date de publication et mention de la délivrance du brevet: **23.04.2008**
(21) Numéro de dépôt: 03753650.5
(22) Date de dépôt: 17.07.2003
(51) Int. Cl.: A61F 9/008

(54) **APPAREIL POUR LE TRAITEMENT DE LA DEGENERESCENCE MACULAIRE LIEE A L'AGE (DMLA)**
VORRICHTUNG ZUM BEHANDELN ALTERSBEDINGTER MAKULADEGENERATION
APPARATUS FOR TREATING AGE-RELATED MACULOPATHY (ARM)

(30) Priorité: 18.07.2002 FR 0209132
(43) Date de publication de la demande: 20.04.2005
(73) Titulaire: UNIVERSITE DES SCIENCES ET TECHNOLOGIES DE LILLE, 59655 Villeneuve-d'Ascq Cedex (FR)
(72) Inventeur: ZEMMOURI, Jaouad, F-59510 Hem (FR); RAZDOBREEV, Igor, F-59155 Faches-Thumesnil (FR); KURKOV, Andrey, F-109180 Mouscou (RU)
(74) Mandataire: Hennion, Jean-Claude
(86) Numéro de dépôt international: PCT/FR2003/002259
(87) Numéro de publication internationale: WO 2004/009005

(56) Documents cités:
- WO-A-01/83030
- WO-A-02/45633
- WO-A-98/40006
- US-A- 5 226 903
- US-A- 6 162 242

## Description

La présente invention concerne un appareil utilisé en ophtalmologie pour traiter au moyen d'un faisceau lumineux, et plus particulièrement d'un laser « non thermique », la dégénérescence maculaire liée à l'âge (DMLA), et plus particulièrement la DMLA de type exsudative.

La dégénérescence maculaire liée à l'âge (DMLA) constitue une menace sévère pour la vision, la fréquence des cas de DMLA tendant à augmenter du fait de l'allongement de durée de vie et de différents facteurs environnementaux et d'exposition quotidienne à la lumière, lesquels facteurs viennent s'ajouter aux prédispositions génétiques.

La DMLA se traduit par la perte progressive des cellules visuelles maculaires. Première cause de mauvaise vision dans les pays industrialisés chez les personnes de plus de cinquante ans, elle n'atteint que la macula. Aussi, elle ne peut être responsable de cécité complète.

Il existe deux formes principales de DMLA :

La DMLA sèche ou atrophique qui se caractérise par une formation de petites tâches ou « drusen » sous la rétine et une atrophie de l'épithélium pigmentaire rétinien. Il n'existe pas de traitement notamment au laser de ce type de DMLA.

La DMLA humide ou exsudative se caractérise par la présence de vaisseaux sanguins anormaux ou néovaisseaux. Ces vaisseaux sont fragiles, peuvent saigner, présenter des fuites, se développer et détruire progressivement la macula. Leur mise en évidence nécessite le plus souvent une angiographie qui les visualise précisément. L'angiographie en fluorescéine montre les fuites de fluides et les néovaisseaux rétiniens et l'angiographie en vert d'indocyanine montre les néovaisseaux choroïdiens et occultes. L'angiographie consiste à injecter du colorant dans les veines afin de mieux observer les vaisseaux sanguins rétiniens et choroïdiens et photographier la rétine après des délais variables.

Les premiers signes de la maladie ne sont visibles que par l'ophtalmologiste et sont dépistés par un examen du fond de l'oeil complété éventuellement par une angiographie à la fluorescéine ou au vert d'indocyanine. Le patient ne perçoit les symptômes qu'à un stade déjà évolué de la maladie. Il n'y a aucune douleur.

Le signe le plus commun est la baisse de l'acuité visuelle ; d'abord sous la forme d'une augmentation de besoin de lumière à la lecture. On voit alors moins de détails, certains traits du visage disparaissent ou certaines lettres ou mots dans une phrase lors de la lecture. Un autre symptôme doit alarmer et décider à consulter rapidement: c'est l'apparition des lignes droites qui deviennent déformées.

Lors d'un stade plus avancé, la vision centrale est très altérée (tâche noire au centre du champ de vision) : les visages ne sont plus reconnus, la lecture et l'écriture deviennent impossibles. La vision périphérique est conservée permettant au patient de se déplacer et de rester autonome.

Différents traitement sont proposés aux patients atteints de la DMLA. Les indications thérapeutiques ne peuvent être précisées davantage, dans la mesure où de nombreux traitements sont en cours d'évaluation. De plus, aucun de ces traitements ne permet de guérir la DMLA. Il n'est possible que de stopper la dégradation ou de ralentir le processus d'évolution de la maladie.

On distingue les traitements de type thermique, dans lesquels un effet de chauffage est recherché et utilisé, des autres traitements.

Les deux traitements de type thermique sont le traitement au laser par photocoagulation, et la thérapie transpupillaire thermique.

### Traitement au laser par photocoagulation :

C'est le premier traitement à avoir fait la preuve de son efficacité dans une forme clinique de néovaisseaux ; néovaisseaux visibles, extra ou juxtafovéolaires.

Il s'agit d'un traitement de réalisation délicate. Le but du traitement est de brûler les néovaisseaux en faisant dégager une forte chaleur à proximité de ceux-ci. Plus précisément, le faisceau cohérent du laser interagit avec la partie arrière de la rétine (l'épithélium pigmentaire) qui absorbe la lumière. Cette réaction va entraîner un dégagement de chaleur en arrière de l'épithélium, ce qui entraîne aussi de manière préjudiciable une lésion définitive des photo-détecteurs.

### La thérapie transpupillaire thermique (TTT) :

La thérapie transpupillaire thermique est un traitement qui consiste à utiliser un laser pour chauffer la zone à traiter comme dans le traitement au laser par photocoagulation. Par contre, dans ce traitement, on utilise le faisceau laser de façon à ce qu'il chauffe moins fort qu'avec un traitement par photocoagulation classique. On tente ainsi d'éviter les lésions mentionnées plus haut dues à l'utilisation d'un laser dégageant une forte chaleur. Cependant, pour obtenir un effet thérapeutique tout en chauffant moins fort, il faut appliquer le faisceau laser sur une plus grande durée (plusieurs dizaines de secondes). C'est ce que décrit la demande de brevet internationale WO 02/45633. En particulier, la durée d'irradiation préconisée avec ce traitement varie de 30 à 40 secondes. Or, tout traitement au laser est un traitement de précision qui impose que la zone à traiter soit fixe pendant la durée du traitement. Maintenir l'oeil d'un patient fixe pendant plusieurs dizaines de secondes est irréalisable, à moins d'appliquer le traitement sous anesthésie.

Par ailleurs, les deux traitements de type non thermique sont les traitements médicamenteux classiques et la photothérapie dynamique.

### Traitements médicamenteux :

Actuellement, aucun traitement médicamenteux n'a encore fait ses preuves. Les traitements les plus étudiés sont ceux utilisant un apport supplémentaire en oligo-éléments et vitamines : vitamine A, E, sélénium, zinc...Le but est de placer la rétine dans de meilleurs conditions métaboliques de fonctionnement et donc de limiter le risque d'accumulation de déchets liés au vieillissement.

### La photothérapie dynamique (PDT) :

La photothérapie dynamique est une méthode récente, qui consiste à combiner un médicament photosensible et un laser « non-thermique » (ne brûlant pas la rétine), par opposition aux lasers utilisés en photocoagulation.

Le médicament photosensible, tel que par exemple celui commercialisé sous la marque Visudyne ®, est injecté par intraveineuse dans le corps du patient. Ce médicament atteint rapidement les vaisseaux sanguins anormaux de la rétine, et se fixe alors sur les parois internes de ces néovaisseaux. On éclaire ensuite la partie de la macula à traiter avec un laser rouge, par exemple à une longueur d'ondes de 689 nm, et pendant une durée de 90 secondes. Ce faisceau laser active alors le médicament photosensible, qui va entraîner une suite de réactions chimiques dans les néovaisseaux, aboutissant à une occlusion des vaisseaux rétiniens anormaux qui vont disparaître par la suite. Plus particulièrement, l'action du faisceau laser sur les molécules du produit photosensible permet principalement de générer de l'oxygène singulet («singlet») (¹O₂), qui est l'agent principal permettant d'obtenir l'occlusion des vaisseaux rétiniens anormaux.

Les dernières études ont démontré des résultats très satisfaisants, avec 60 % des patients traités présentant une acuité visuelle stabilisée ou améliorée. Toutefois, cette méthode présente plusieurs inconvénients. Le premier inconvénient est lié à la photosensibilisation des patients, qui les oblige à éviter toute exposition solaire pendant une durée relativement longue, généralement de l'ordre de 48 h. Un autre inconvénient est lié à l'injection d'un médicament (produit photosensible) qui est onéreux, ce qui rend ce traitement coûteux, étant précisé que le traitement doit être répété pour être efficace. Enfin, l'injection d'un médicament photosensible peut, chez certains patients, entraîner des effets secondaires préjudiciables.

Un objectif de la présente invention est donc de proposer un appareil et une méthode de traitement de la DMLA et en particulier de la DMLA humide qui de manière comparable à la photothérapie dynamique utilise un faisceau lumineux thérapeutique non thermique et non destructif (contrairement au laser utilisé en photocoagulation ou dans la thérapie transpupillaire thermique), mais qui ne nécessite pas l'injection d'un médicament photosensible.

La solution de l'invention repose sur l'utilisation d'une source laser présentant une longueur d'onde d'émission comprise entre 1,26 µm 1,27 µm.

L'invention a ainsi pour objet un appareil pour le traitement par laser de la dégénérescence maculaire liée à l'âge comportant, de manière connue en soi, une source de lumière qui en fonctionnement permet l'émission d'un faisceau lumineux thérapeutique non thermique.

De manière caractéristique selon l'invention, ladite source de lumière est conçue pour émettre un faisceau lumineux thérapeutique non thermique présentant une longueur d'onde d'émission comprise entre 1,26 µm 1,27 µm, de façon à se situer dans une gamme de longueur d'onde correspondant à la transition moléculaire précise de l'oxygène, permettant ainsi de générer directement et en quantité suffisante de l'oxygène singulet en intracellulaire.

L'invention a également pour objet une méthode de traitement de la dégénérescence maculaire liée à l'âge consistant à éclairer la macula avec un faisceau lumineux thérapeutique non thermique présentant une longueur d'onde d'émission comprise entre 1,26 µm - 1,27 µm, de façon à se situer dans une gamme de longueur d'onde correspondant à la transition moléculaire précise de l'oxygène, permettant ainsi de générer directement et en quantité suffisante de l'oxygène singulet en intracellulaire.

De préférence la source de lumière est une source laser (faisceau lumineux thérapeutique cohérent).

De préférence, mais de manière non limitative de l'invention, la source laser utilisée est un laser Raman à fibre.

Plus particulièrement, la puissance de la source laser est comprise entre 1 mW et 1 W, et de préférence comprise entre 10 mW et 1 W.

Il, a été constaté que l'utilisation d'un faisceau lumineux thérapeutique aux fréquences particulières précitées permettait avantageusement et de manière surprenante, d'obtenir des résultats satisfaisants en ce qui concerne le traitement de la DMLA (retardement de la perte d'acuité visuelle, et voire même amélioration de l'acuité visuelle) sans qu'il soit nécessaire d'utiliser un médicament. A posteriori, on peut supposer que l'action de ce faisceau lumineux thérapeutique dans la gamme de longueur d'ondes précitée permettrait de générer de l'oxygène singulet, directement à partir du sang contenu dans les néovaisseaux, et ce en quantité suffisante pour obtenir des effets thérapeutiques comparables à ceux obtenus avec l'injection d'un médicament photosensible. Ainsi, contrairement à la méthode PDT précitée, le faisceau lumineux n'agit pas sur un produit intermédiaire (médicament photosensible) pour générer l'oxygène singulet, mais compte tenu de la gamme de longueur d'ondes particulière précitée du faisceaux lumineux utilisé dans l'invention, ledit faiceau agit directement sur l'oxygène contenu dans les néovaisseaux pour générer l'oxygène singulet. La demanderesse n'est toutefois pas liée par cette explication.

D'autres caractéristiques et avantages de l'invention apparaîtront plus clairement à la lumière de la description ci-après d'une variante préférée de réalisation d'un appareil de traitement de l'invention et de son utilisation, laquelle description est donnée à titre d'exemple non limitatif et en référence aux dessins annexés sur lesquels :
- la figure 1 représente un exemple d'appareil de traitement de l'invention, de type lampe à fente,
- la figure 2 est un schéma détaillant les deux principales sources lumineuses (source thérapeutique et source d'éclairage) montées à l'intérieur de l'unité de traitement de l'appareil de la figure 1,
- la figure 3 est un schéma d'un laser Raman à fibre mis en oeuvre dans l'appareil de la figure 1.

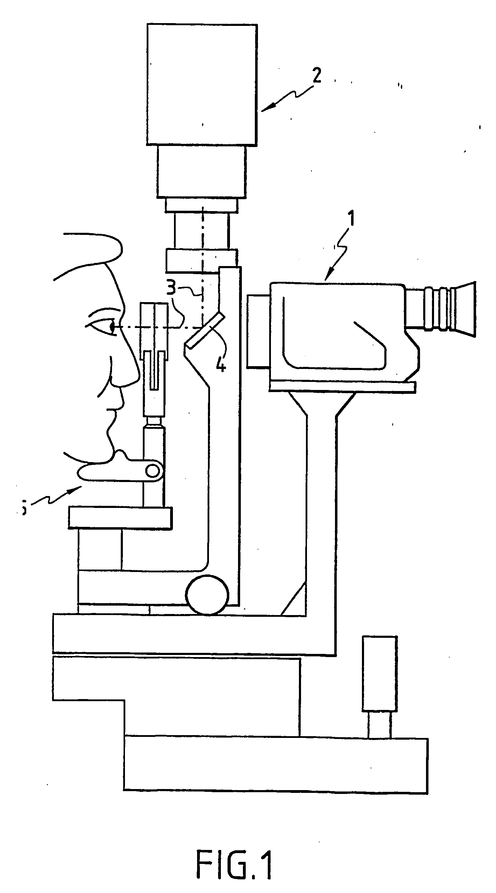

On a représenté sur la figure 1, un exemple de réalisation d'un d'appareil de l'invention, qui permet de traiter la DMLA et qui est de type lampe à fente.

Cet appareil comporte essentiellement, et de manière usuelle dans le domaine des lampes à fente :
- une unité optique 1 pour l'observation par le praticien de l'oeil à traiter,
- une unité de traitement 2 qui permet de générer un faisceau lumineux 3, lequel faisceau est en l'occurrence orienté verticalement en sortie de l'unité de traitement 2,
- un miroir 4 permettant de dévier à 90° le faisceau lumineux 3, de telle sorte que celui-ci est orienté horizontalement selon le même axe que l'axe optique d'observation de l'unité optique 2 ;
- des moyens 5, de type mentonnière, permettant de positionner la tête du patient, et par là même l'oeil à traiter, par rapport à l'unité optique 1 et au faisceau lumineux 3.

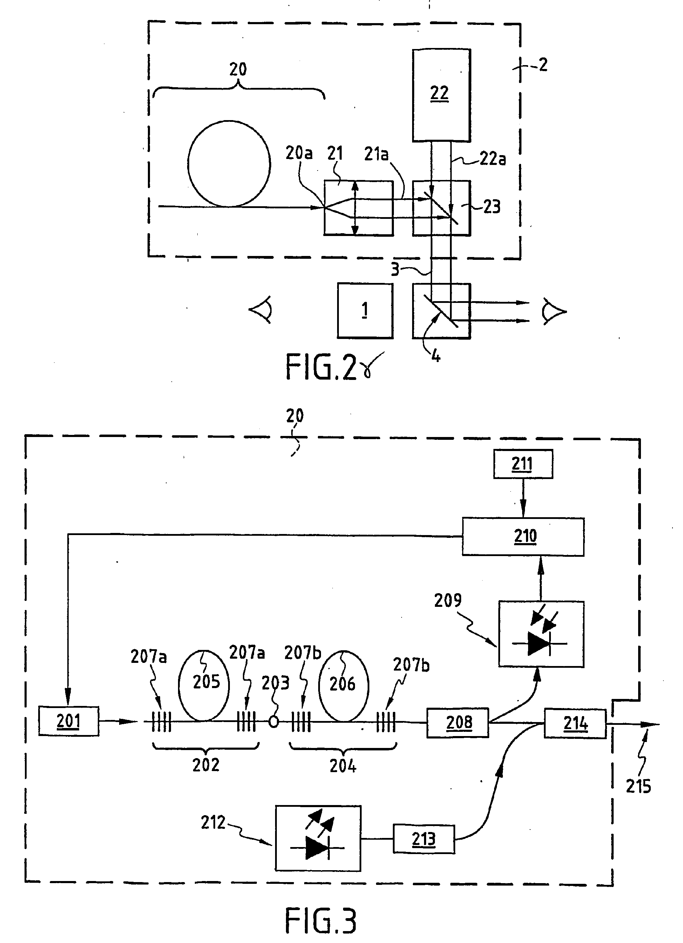

En référence à la figure 2, l'unité de traitement 2 intègre une source lumineuse thérapeutique 20 et une source lumineuse 22 qui est usuelle en soi et permet l'éclairage de l'oeil (lampe d'éclairage standard). Le faisceau lumineux thérapeutique 20a issu de la source 20 est couplé de manière usuelle avec le faisceau éclairant 22a issu de la source lumineuse 22 par un système optique de couplage 23, après avoir été mis en forme de manière également connue (système optique de collimation 21). En fonctionnement, en sortie de l'unité de traitement 2, est émis le faisceau lumineux 3 précité, qui comprend à la fois un faisceau de lumière thérapeutique et un faisceau de lumière non thérapeutique permettant l'éclairage de l'oeil en vue de l'observation de l'oeil au moyen de l'unité optique 1.

L'invention réside dans la source lumineuse 20 de l'unité 2 qui va a présent être détaillée. Les autres éléments de l'appareil étant ceux usuellement mis en oeuvre dans les lampes à fente de l'art antérieur, ils ne seront pas plus amplement décrits dans la suite de la description.

D'une manière générale selon l'invention, la source lumineuse 20 est conçue pour émettre un faisceau de lumière thérapeutique 20a présentant une longueur d'onde d'émission comprise en 1,2µm et 1,3µm.

De préférence, le faisceau de lumière thérapeutique 20a est un faisceau de lumière cohérente (laser). Néanmoins, dans une autre réalisation, le faisceau de lumière thérapeutique 20a pourrait être un faisceau de lumière incohérente, généré à partir d'une source lumineuse de puissance suffisante suivi d'un filtrage optique pour ne conserver que les composantes fréquentielles dans la gamme 1,2µm et 1,3µm.

S'agissant d'un faisceau 20a de lumière cohérente, dans sa portée la plus générale, l'invention n'est pas limitée à un type particulier de source laser 20, toute source laser permettant l'émission d'un faisceau laser remplissant la condition de longueur d'onde ci-dessus, et connue de l'homme du métier, pouvant être utilisé. En particulier, et de manière non exhaustive, on peut utiliser les types de source laser suivants :
- Laser raman à fibre continu ou impulsionnel ;
- Laser Cr : Forsterite (Cr₄+ : Mg₂SiO₄) pulsé ou continu, pompé par un laser solide ou à fibre dopé néodyme (Nd), par un laser solide ou à fibre dopé Ytterbium, ou pompé par diode ;
- Oscillateur paramétrique pulsé ou continu, pompé par une autre source laser,
- Diode laser,
- Laser ou convertisseur Raman solide continu ou impulsionnel pompé par une autre source laser.

Parmi les lasers ci-dessus, on utilise de préférence un laser Raman à fibre pour les raisons principales suivantes :
- la sortie fibrée du laser facilite le transport du faisceau jusqu'à la sortie de l'unité de traitement 2 ;
- le faisceau laser 20a généré présente une bonne qualité spectrale et spatiale ;
- la source laser 20 est avantageusement compacte,
- la source laser 20 est fiable et ne nécessite aucune maintenance ;
- ce type de source laser offre le meilleur compromis dualité/ coût de fabrication du laser.

### Exemple préféré de réalisation d'un laser Raman à fibre à une longueur d'onde comprise entre 1,2µm et 1,3µm (Figure 3)

La source laser 20 utilisée dans l'unité de traitement 2 est de préférence un laser Raman à fibre, conçu pour émettre un faisceau laser 20a thérapeutique à une longueur d'onde de 1,26µm-1,27µm, avec une puissance comprise entre 1 mW et 1 W, et de préférence comprise entre 10 mW et 1 W.

En référence à la figure 3, ce laser Raman à fibre comporte une diode laser de pompe 201 à une longueur d'onde de 910-930 nm ou 970-980 nm, un laser à fibre dopée Ytterbium Yb 202, et un convertisseur Raman 204 qui a pour fonction de transposer la longueur d'onde du faisceau en sortie du laser à fibre 202, en sorte d'obtenir un faisceau laser à la longueur d'onde 1260nm-1270nm.

Le laser à fibre dopée Yb 202 est constitué d'une fibre à double gaine 205 dont le coeur est dopé en Ytterbium et de deux réseaux de Bragg 207a en entrée et en sortie qui sont photo-inscrits dans la fibre. La sortie 203 de la fibre du laser Yb 202 est soudée directement à l'entrée du convertisseur Raman 204.

Le convertisseur Raman 204 comprend une fibre 206 dont le coeur est dopé en phosphore et deux réseaux de Bragg (207b) en entrée et en sortie qui sont réglés à une longueur d'onde dans la gamme 1260 - 1270 nm. Ce convertisseur permet d'effectuer la transposition de la longueur d'émission du laser Yb (202) en un seul pas.

Dans une autre variante, Il est possible d'utiliser une fibre monomode ou mulitimode, différente de la fibre précédente ; il convient dans ce cas d'adapter le nombre de pas de conversion du convertisseur Raman 204 en fonction de la nature de la fibre, et notamment du type de dopant utilisé.

Il est possible également de remplacer les réseaux de Bragg par des coupleurs monomodes.

Le contrôle de puissance s'effectue via un coupleur 208 présentant un faible taux de couplage, et une photodiode 209 reliée à une électronique de contrôle 210, laquelle permet de contrôler la puissance en sortie 215, en agissant, par exemple directement sur le courant de la diode de pompe 201. L'électronique de contrôle 210 permet au praticien de régler manuellement la puissance du faisceau laser thérapeutique 20a, entre 1 mW et 1 W, et de préférence entre 10 mW et 1 W.

La sortie 215 du laser Raman à fibre est par exemple équipée d'un connecteur de sortie non représenté sur les figures (connecteur de type FC, SMA ou autre), permettant de la relier facilement et directement à l'optique de collimation 21 de la lampe à fente.

Une pédale d'action 211 ou équivalent reliée à l'électronique de contrôle 210 est également prévue pour permettre au praticien de commander le déclenchement du faisceau laser thérapeutique 20a.

Selon une caractéristique additionnelle, la source laser 20 intègre également des moyens de visée (212,213,214). Plus particulièrement, ces moyens de visée comportent une diode laser rouge fibrée 212 qui émet dans la gamme des longueurs d'onde 630 - 690 nm, et qui est couplée à la sortie du laser Raman à fibre via un atténuateur 213 et un coupleur WDM 214 monomode. La diode 212 sert de source de visée, et permet au praticien (via l'unité optique 1) de visualiser le point d'impact du faisceau laser thérapeutique.

Le laser Raman à fibres qui vient d'être décrit en référence à la figure 3, et qui permet l'émission d'un faisceau laser thérapeutique à une longueur d'onde comprise entre 1,2 µm et 1,3 µm est nouveau en soi, et peut donc avantageusement également être utilisé dans d'autres applications médicales ou non, en dehors du domaine particulier du traitement de la DMLA.

La mise en oeuvre de l'appareil de l'invention est la suivante. Le patient positionne sa tête sur la mentonnière 5. Le praticien règle de manière très précise et connue en soi la position spatiale de l'oeil à traiter par rapport au faisceau 3, en visualisant l'impact du faisceau laser thérapeutique 20a au moyen du faisceau de visée produit continuellement par la diode 212. Lorsque l'alignement est parfait, le praticien règle la puissance d'émission du faisceau thérapeutique 20a, et actionne la pédale ou équivalent de commande 211, pendant une durée prédéterminée, ce qui déclenche l'émission du faisceau thérapeutique (éclairage de la zone à traiter de la macula). La durée de traitement est calculée par le praticien. Eventuellement, des moyen électroniques de contrôle de cette durée de traitement peuvent être prévus sur l'appareil. Lorsque la zone visée de la macula est traitée, le praticien réitère ces opérations sur une nouvelle zone malade (nouvel alignement de l'oeil par rapport au faisceau 3).

## Revendications

1. Appareil pour le traitement de la dégénérescence maculaire liée à l'âge, comportant une source de lumière qui en fonctionnement permet l'émission d'un faisceau lumineux thérapeutique de façon similaire à la source de lumière utilisée dans le cadre de la photothérapie dynamique, **caractérisé en ce que** ladite source de lumière est conçue pour émettre un faisceau lumineux thérapeutique (20a) présentant une longueur d'onde d'émission comprise entre 1,26 µm et 1,27 µm, de façon à générer de l'oxygène singulet en intracellulaire directement et en quantité suffisante.

2. Appareil selon la revendication 1 **caractérisé en ce que** la puissance du faisceau lumineux thérapeutique (20a) est comprise entre 1mW et 1W, et de préférence entre 10mW et 1W.

3. Appareil selon l'une des revendications 1 à 2 **caractérisé en ce que** la source de lumière thérapeutique est une source laser.

4. Appareil selon la revendication 3 **caractérisé en ce que** la source laser comporte un laser Raman à fibre (20).

5. Appareil selon la revendication 4 **caractérisé en ce que** le laser Raman à fibre comprend une diode laser de pompe (201), un laser à fibre dopée Ytterbium (202), et un convertisseur Raman (204) qui a pour fonction de transposer la longueur d'onde du faisceau issu du laser à fibre doper Ytterbium (202).

## Claims

1. Apparatus for treating age-related macular degeneration, the apparatus comprising a light source which, in operation, enables a therapeutic light beam to be emitted in a manner similar to the light source used in the context of dynamic phototherapy, the apparatus being **characterized in that** said light source is designed to emit a therapeutic light beam (20a) presenting an emission wavelength lying in the range 1.26 µm to 1.27 µm, thereby generating intracellular singlet oxygen directly and in sufficient quantity.

2. Apparatus according to claim 1, **characterized in that** the power of the therapeutic light beam (20a) lies in the range 1 mW to 1 W, and preferably in the range 10 mW to 1 W.

3. Apparatus according to claim 1 or claim 2, **characterized in that** the therapeutic light source is a laser source.

4. Apparatus according to claim 3, **characterized in that** the laser source comprises an optical fiber Raman laser (20).

5. Apparatus according to claim 4, **characterized in that** the optical fiber Raman laser comprises a pump laser diode (201), an ytterbium-doped optical fiber laser (202), and a Raman converter (204) serving to transpose the wavelength of the beam coming from the ytterbium-doped optical fiber laser (202).

## Patentansprüche

1. Gerät für die Behandlung der altersbedingten Makula-Degeneration, umfassend eine Lichtquelle, die im Betrieb das Aussenden eines therapeutischen Lichtstrahls ähnlich der Lichtquelle, die im Rahmen der dynamischen Phototherapie verwendet wird, ermöglicht, **dadurch gekennzeichnet, daß** die genannte Lichtquelle ausgelegt ist, um einen therapeutischen Lichtstrahl (20a) auszusenden, der eine Sendewellenlänge zwischen 1,26 µm und 1,27 µm aufweist, so daß Singulett-Sauerstoff direkt und in ausreichender Menge intrazellulär erzeugt wird.

2. Gerät nach Anspruch 1, **dadurch gekennzeichnet, daß** die Leistung des therapeutischen Lichtstrahls (20a) zwischen 1 mW und 1 W und vorzugsweise zwischen 10 mW und 1 W liegt.

3. Gerät nach einem der Ansprüche 1 bis 2, **dadurch gekennzeichnet, daß** die therapeutische Lichtquelle eine Laserquelle ist.

4. Gerät nach Anspruch 3, **dadurch gekennzeichnet, daß** die Laserquelle einen Faser-Raman-Laser (20) aufweist.

5. Gerät nach Anspruch 4, **dadurch gekennzeichnet, daß** der Faser-Raman-Laser eine Pump-Laserdiode (201), einen Ytterbium-Faserlaser (202) und einen Raman-Konverter (204) aufweist, dessen Aufgabe darin besteht, die Wellenlänge des aus dem Ytterbium-Faserlaser (202) ausgetretenen Strahls umzusetzen.
